# EUROPÄISCHE PATENTSCHRIFT

(11) **EP 0 475 003 B1**
(45) Veröffentlichungstag und Bekanntmachung des Hinweises auf die Patenterteilung: **04.11.1998**
(21) Anmeldenummer: 91111102.9
(22) Anmeldetag: 04.07.1991
(51) Int. Cl.: C07D 211/74, C07D 211/46, C07D 211/58, C07D 211/16, C07D 401/14, C07D 401/12, C08G 18/80, C08G 18/10, C08G 18/28, C08G 18/79, C08G 18/75, C08G 18/32

(54) **Blockierte (cyclo)-aliphatische Polyisocyanate sowie ein Verfahren zu deren Herstellung**
Blocked (cyclo)aliphatic polyisocyanates and process for their preparation
Polyisocyanates (cyclo)aliphatiques bloqués et procédé de leur préparation

(30) Priorität: 06.09.1990 DE 4028285
(43) Veröffentlichungstag der Anmeldung: 18.03.1992
(73) Patentinhaber: HÜLS AKTIENGESELLSCHAFT, 45764 Marl (DE)
(72) Erfinder: Gras, Rainer, Dr., W-4630 Bochum 5 (DE); Wolf, Elmar, Dr., W-4350 Recklinghausen (DE)

(56) Entgegenhaltungen:
- EP-A- 0 096 210
- FR-A- 2 364 935
- US-A- 4 243 792
- FATIPEC-Kongressbuch 1980, II,S. 293-306

## Beschreibung

Die vorliegende Erfindung betrifft neue blockierte (cyclo)-aliphatische Polyisocyanate sowie ein Verfahren zu deren Herstellung.

Es ist bekannt, daß Polyamine mit Polyisocyanaten so schnell reagieren, daß sie nicht verarbeitbar sind, wenn duroplastische PUR-Massen gebildet werden. Dagegen lassen sich Diamine mit Diisocyanaten zu thermoplastischen PUR-Massen entweder in Lösung oder in der Schmelze verarbeiten. Bei den zuletzt genannten thermoplastisch verarbeitbaren PUR-Systemen werden durch Einführung von Harnstoffsegmenten die Eigenschaften der PUR-Elastomeren, vor allem Schlagzähigkeit, Zugfestigkeit und Weiterreißfestigkeit, verbessert.

Es ist also nicht möglich, vernetzte PUR-Systeme, z. B. durch Reaktion eines Diamins mit einem Triisocyanat, oder umgekehrt durch Reaktion eines Triamins mit einem Diisocyanat, herzustellen. In der DE-OS 10 86 372 werden erstmals Überzugsmittel bzw. Reaktionslacke vorgestellt, deren Herstellung bei Raumtemperatur durch Reaktion eines Phenol-blockierten aromatischen Triisocyanats und eines Amidgruppen enthaltenden Diamins erfolgt: 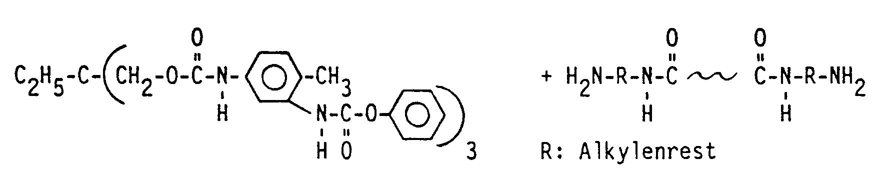 Voraussetzung für dieses Prinzip - Verminderung der NCO-Reaktivität gegenüber NH₂-Gruppen durch Blockierung der NCO-Gruppen - ist, daß das Blockierungsmittel nur so fest gebunden ist, daß es bereits bei Raumtemperatur durch Amine wieder verdrängt werden kann, wie dies ja für die Phenol-blockierten aromatischen NCO-Gruppen zutrifft. Am Fatipec-Kongreß 1980 (Fatipec-Kongreßbuch II, 293-306) wurden flexible, vernetzte PUR-Elastomere vorgestellt, die nach dem gleichen Prinzip - Verminderung der NCO-Reaktivität durch Blockierung - hergestellt wurden. Bei diesen vernetzten PUR-Elastomeren handelt es sich um ein polymeres Netzwerk, das durch Reaktion eines Nonylphenol-blockierten Isocyanatadduktes aus 1 Mol Polypropylenethertriol (MG: ca. 3 000) und 3 Molen Toluylendiisocyanat mit einem Diamin, z. B. LAROMIN® C 260 der Firma BASF (3.3'-Dimethyl-4.4'-diaminodicyclohexylmethan) bei Raumtemperatur im NCO:NH₂-Verhältnis von 1 : 1 erhalten wird.

Beiden, durch eine NCO/NH₂-Reaktion vernetzten PUR-Systemen haftet der Nachteil an, daß sie nicht lichtbeständig sind, da es sich jeweils um aromatische Polyurethane handelt, die ja bekanntermaßen bei Bewitterung zur Verfärbung neigen.

Diesen Nachteil der aromatischen PUR-Systeme durch einfache Substitution des aromatischen Isocyanats durch ein (cylco)-aliphatisches zu beseitigen - aliphatische Polyurethane zeigen hervorragende Lichtbeständigkeit und Wetterstabilität - ist nicht möglich, da Phenol-blockierte (cyclo)-aliphatische NCO-Gruppen bei Raumtemperatur mit Aminogruppen nicht reagieren.

In der DE-OS 28 20 419 wird ein feuchtaushärtender PUR-Decklack mit verbesserter Glanzbeständigkeit offenbart. Diese verbesserte Glanzbeständigkeit wird durch Einbau von geringen Mengen (ca. 1 %) einer HALS-Verbindung (TINUVIN 770) erreicht.

In der DE-OS 26 42 374 werden permanent stabilisierte Polyurethane beschrieben. Hier werden die HALS-Verbindungen durch eine reaktive Gruppe in 4-Stellung des 2.2.6.6-Tetramethylpiperidin-Rings in das NCO-Präpolymere eingebaut.

In der EP 0 096 210 werden lagerstabile PUR-Einkomponenten-Einbrennlacke offenbart, die oberhalb 120 °C aushärten. Als Härter werden mit sterisch gehinderten Aminen blockierte (cyclo)aliphatische Polyisocyanate beschrieben. Diese blockierten Polyisocyanate lassen sich mit einer Ausnahme bei Raumtemperatur nicht mit (cyclo)aliphatischen Polyaminen umsetzen. Bei dieser Ausnahme handelt es sich um das mit 2.2.6.6-Tetramethylpiperidin blockierte IPDI.

Aufgabe der vorliegenden Erfindung ist somit das Auffinden von blockierten (cyclo)aliphatischen Polyisocyanaten, die bei Raumtemperatur mit Aminen unter Freisetzung des Blockierungsmittels reagieren.

Überraschenderweise konnte diese Aufgabe dadurch gelöst werden, daß die (cyclo)aliphatischen Polyisocyanate mit speziellen Piperidin-Derivaten blockiert werden.

Gegenstand der Erfindung sind daher blockierte aliphatische, cycloaliphatische, araliphatische oder arylsubstituierte aliphatische Polyisocyanate, wobei diese ggf. mit Polyolen kettenverlängert sein können und die Polyisocyanate auch Uretdion-, Carbodiimid-, Harnstoff- und Isocyanurat- bzw. Biuretgruppen aufweisen können, enthaltend als Blockierungsmittel sterisch gehinderte Piperidin-Derivate folgender Formeln A, B oder C 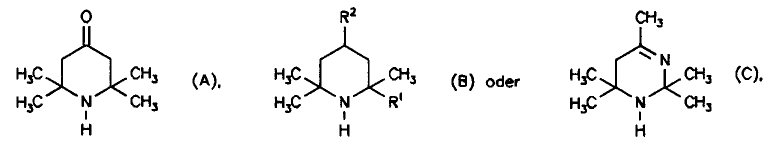 wobei
R¹ = H, CH₃,
R² = (-O-)ₙR³, (-O-CO)ₙ-R³, -OH , -NH-CO-R³, -N-(CH₃)₂ , 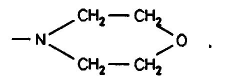 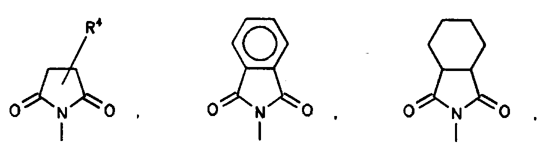
n = 1,2
R³ = C₁-C₁₈-Alkyl, wenn n = 1 und
R³ = C₂-C₁₈-Alkylen, wenn n = 2
R⁴ = H, C₁-C₂₀-Alkyl bedeuten, und
   wobei das NCO:NH-Verhältnis 0,5 bis 1,4, vorzugsweise 0,9 bis 1,1, insbesondere 1, beträgt.

Die Ether- und Esterverbindungen nach Formel (B) können auch in bifunktioneller Form eingesetzt werden, wie z. B. Bis-(2.2.6.6-Tetramethyl-4-piperidyl)-sebacat.

Als Ausgangsverbindungen, die zur Blockierung mit den sterisch gehinderten Piperidin-Derivaten (A) bzw. (B) eingesetzt werden können, eignen sich beispielsweise Polyisocyanate, insbesondere Diisocyanate, wie aliphatische, (cyclo)-aliphatische, araliphatische, arylsubstituierte aliphatische Diisocyanate, wie sie beispielsweise in Houben-Weyl, Methoden der organischen Chemie, Band 14/2, S. 61-70 und dem Artikel von W. Siefken in Justus Liebigs Annalen der Chemie 562, 75-136, beschrieben werden.

Neben den monomeren Polyisocyanaten können als Ausgangsstoffe für die Blockierung mit den nachstehend ausführlich beschriebenen sterisch gehinderten Piperidinen selbstverständlich auch die dimeren und trimeren Formen der Polyisocyanate, wie Uretdione, Carbodimide, Harnstoffe und Isocyanurate bzw. Biurete, eingesetzt werden, die nach bekannten Methoden herstellbar sind.

Unter Polyisocyanaten im Sinne der vorliegenden Erfindung werden auch solche verstanden, die vor der Blockierung mit den sterisch gehinderten Piperidin-Derivaten einer Umsetzung zur Molekülvergrößerung mit den in der Isocyanatchemie gebräuchlichen Kettenverlängerungsmitteln, wie z. B. Polyolen, unterworfen wurden, wobei das bi- oder höherfunktionelle Kettenverlängerungsmittel - also das Polyol - in solchen Mengen verwendet wird, daß das resultierende neue Isocyanat im Durchschnitt mindestens 2 Isocyanatgruppen trägt.

Geeignete Polyole sind beispielsweise Diole und Triole, wie z. B. Ethylenglykol, 1.2- und 1.3-Propandiol 2.2-Dimethylpropandiol-1.3, Butandiol-1.4, Hexandiol-1.6, 2.2.4(2.4.4)-Trimethylhexandiol-1.6, Octadecandiol-1.18, Diethylenglykol, Triethylenglykol, trans- und/oder cis-1.4-Cyclohexandimethanol Glycerin, Hexantriol-1.2.6, 1.1.1-Trimethylolpropan, 1.1.1-Trimethylolethan sowie Polyolgemische.

Bei den zur Kettenverlängerung eingesetzten Polyolen kann es sich auch um Polyester- und/oder Polyetherpolyole handeln. Die Polyesterpolyole sind Kondensationsprodukte aus Dicarbonsäuren und Polyolen, die durch Polykondensation, z. B. von Adipinsäure, 2.2.4-(2.4.4)-Trimethyladipinsäure, Phthalsäure, Isophthalsäure, Terephthalsäure sowie deren hydrierte Formen mit Ethylenglykol, Butandiol, Diethylenglykol, Triethylenglykol, Hexandiol, Neopentylglykol, 1.1.1-Trimethylolpropan, hergestellt werden. Ferner können die Polyesterpolyole auch Polymerisationsprodukte des Caprolactons sein. Aber auch OH-Gruppen enthaltende Polymerisationsprodukte werden als Kettenverlängerungsmittel für die genannten Diisocyanate eingesetzt. Es sind dies z. B. Polyalkylenetherpolyole, die durch anionische Polymerisation, Copolymerisation und Blockcopolymerisation von Ethylenoxiden, wie Ethylenoxid, Propylenoxid, Butylenoxid mit bi- oder polyfunktionellen Alkoholen, wie Butandiol, 1.1.1-Trimethylolpropan, Hexantriol-1.2.6, Pentaerythrit, oder mit Aminen, wie Methylamin, Ethylendiamin als Starterkomponente, oder anionische Polymerisation und Copolymerisation cyclischer Ether, wie Tetrahydrofuran, Ethylenoxid und Propylenoxid, mit sauren Katalysatoren, wie z. B. Bortrifluoridetherat, hergestellt werden.

Geeignete sterisch gehinderte Piperidin-Derivate im Sinne der vorliegenden Erfindung, die den Formeln (A) bzw. (B) entsprechen, sind beispielsweise 2.2.6.6-Tetramethylpiperidinon-4 (Triacetonamin, TAA), 4-Dimethylamino-2.2.6.6-tetramethylpiperidin, Kondensationsprodukte aus 4-Hydroxy- bzw. Amino-2.2.6.6-tetramethylpiperidin und Monocarbonsäuren mit mindestens einem C-Atom, oder Dicarbonsäuren mit mindestens zwei C-Atomen, die aromatische oder cycloaliphatische Ringe bilden können, die gegebenenfalls noch substituiert sind. Des weiteren ist auch Acetonin geeignet.

Ein wesentliches Merkmal der vorliegenden Erfindung ist, daß das Piperidin-Derivat in α-Stellung zum N mindestens 3 Alkyl-Substituenten - in der Regel Methylgruppen - enthält. So ist z. B. 2.2.6-Trimethylpiperidin zur Herstellung der erfindungsgemäßen Verbindungen geeignet, während sich 2.6-Dimethylpiperidin zur Herstellung der erfindungsgemäßen Verbindungen, die bereits bei Raumtemperatur mit Aminen reagieren, als ungeeignet erweist.

Bei den erfindungsgemäßen Verbindungen handelt es sich im allgemeinen um Verbindungen des Molekulargewichtsbereichs von 300 bis 6 000. Je nach zur Blockierung eingesetztem Polyisocyanat und je nach Blockierungsmittel können die erfindungsgemäßen Verbindungen fest oder flüssig sein. Die mit den sterisch gehinderten Piperidin-Derivaten blockierten Polyisocyanate sind durch einen Gehalt an endständig in blockierter Form vorliegenden Isocyanatgruppen (berechnet als NCO) von 1 bis 25 % charakterisiert.

Ein weiterer Gegenstand der vorliegenden Erfindung ist das Verfahren zur Herstellung der erfindungsgemäßen blockierten Polyisocyanate. Die Herstellung der blockierten Polyisocyanate erfolgt durch Umsetzung von Polyisocyanaten mit den sterisch gehinderten Piperidin-Derivaten der allgemeinen Formeln: 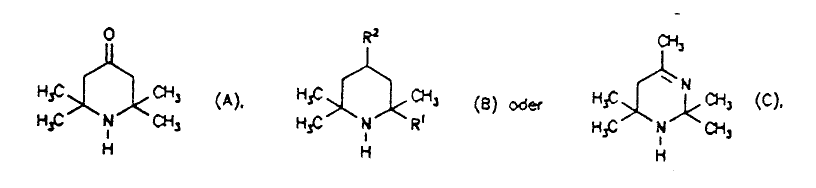 wobei
R¹ = H, CH₃,
R² = (-O-)ₙR³, (-O-CO)ₙ-R³, -OH , -NH-CO-R³, -N-(CH₃)₂ , 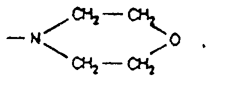 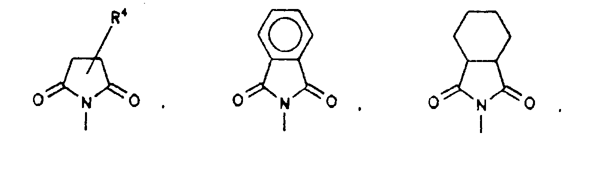
n = 1,2
R³ = C₁-C₁₈-Alkyl, wenn n = 1 und
R³ = C₂-C₁₈-Alkylen, wenn n = 2
R⁴ = H, C₁-C₂₀-Alkyl bedeuten,
   bei 20 bis 100 °C so umgesetzt werden, wobei das NCO:NH-Verhältnis 0,5 bis 1,4, vorzugsweise 0,9 bis 1,1, insbesondere 1, beträgt.

Die Polyisocyanate und die sterisch gehinderten Piperidin-Derivate werden in solchen Mengen eingesetzt, daß auf ein Hol Isocyanatgruppen in der Regel 1 Mol sterisch gehindertes Piperidin kommt. In manchen Fällen hat es sich als zweckmäßig erwiesen, ein NCO:NH-Verhältnis von 0,5 bis 1,4, vorzugsweise von 0,9 bis 1,1, zu wählen.

Die Umsetzung kann sowohl in Lösungsmitteln als auch in der Schmelze durchgeführt werden. Als Lösungsmittel für diese Reaktion kommen nur solche infrage, die mit den Polyisocyanaten nicht reagieren, beispielsweise Ketone, wie Aceton, Methylethylketon, Methylisobutylketon, Cyclopentanon, Cyclohexanon, Aromaten, wie Toluol, Xylol, Chlorbenzol u. a., cyclische Ether, wie Tetrahydrofuran; Ester, wie Methylacetat, n-Butylacetat u. a.; aliphatische Chlorkohlenwasserstoffe, wie Chloroform, Tetrachlorkohlenstoff u. a., sowie aprotische Lösungsmittel, wie Dimethylformamid, Dimethylacetamid, Dimethylsulfoxid usw.. Als besonders vorteilhaft hat es sich erwiesen, die Blockierung in Weichmachern, wie Dicarbonsäureestern, wie z. B. Phthalsäureestern, Phosphorsäureestern, Chlorparaffinen, Sulfonsäureestern, durchzuführen.

Bei der Blockierung des Polyisocyanats geht man so vor, daß das Polyisocyanat und das sterisch gehinderte Piperidin-Derivat so lange bei der angegebenen Temperatur erhitzt werden, bis der NCO-Gehalt des Reaktionsgemisches unter 0,5 % NCO abgesunken ist.

Wird das Polyisocyanat mit einem bifunktionellen Piperidinderivat blockiert, so ist es zweckmäßig, insbesondere bei ≥ 2 funktionellen Polyisocyanaten mindestens 50 Mol-% der NCO-Gruppen mit einem monofunktionellen Piperidin umzusetzen.

Die erfindungsgemäßen Verbindungen eignen sich zur Herstellung von kalthärtenden, hochelastischen Polyurethanmassen.

### Beispiel 1

222 Gew.-T. IPDI wurden auf 80 °C erhitzt. Unter N₂-Abdeckung wurden portionsweise 310 Gew.-T. TAA zudosiert, wobei intensiv gerührt wurde. Nach der TAA-Zugabe wurde noch ca. 5 h bei 80 °C weitererhitzt. Das Reaktionsprodukt hatte einen blockierten NCO-Gehalt von 15,7 % und einen Schmelzbereich von 44 bis 50 °C.

### Beispiel 2

444 Gew.-T. IPDI und 106 Gew.-T. Diethylenglykol wurden bei 80 °C so lange erhitzt, bis der NCO-Gehalt der Reaktionsmischung 15,3 % erreicht hatte. Anschließend wurden unter N₂-Abdeckung 310 Gew.-T. TAA portionsweise zugegeben und so lange bei 80 °C weitererhitzt, bis der NCO-Gehalt < 0,5 % betrug. Das Reaktionsprodukt hatte einen Schmeizbereich von 63 bis 67 °C und einen blockierten NCO-Gehalt von 9,7 %.

### Beispiel 3

210 Gew.-T. 2.2.4(2.4.4)-Trimethylhexamethylendiisocyanat wurden mit 310 Gew.-T. TAA in analoger Weise zum Beispiel 1 umgesetzt. Das Reaktionsprodukt hatte eine Viskosität von 186 000 mPa·s (bei RT); der blockierte NCO-Gehalt betrug 16 %.

### Beispiel 4

243 Gew.-T. des trimeren IPDI (Isocyanurat des IPDI) wurden in einem Gemisch aus 166 Gew.-T. SOLVESSO^{(R)} 100 (Aromatengemisch, Siedebereich 183-181°C)und 99 Gew.-T. Butylacetat gelöst und mit 155 Gew.-T. TAA versetzt. Die Lösung wurde solange bei 80 °C unter N₂-Abdeckung erhitzt, bis der NCO-Gehalt < 0,5 % betrug. Die Viskosität des Reaktionsproduktes betrug 1 130 mPa·s (bei RT), der blockierte NCO-Gehalt betrug 6,4 %.

### Beispiel 5

168 Gew.-T. 2-Methylpentamethylendiisocyanat-1.5 (mit einem Gehalt von 4,2 % 2-Ethyltetramethylendiisocyanat-1.4) wurden in Analogie zum Beispiel 1 mit 155 Gew.-T. TAA umgesetzt. Das Reaktionsprodukt hatte (bei RT) eine Viskosität von 74 000 mPas und Gesamt-NCO-Gehalt (blockiertes + freies NCO) von 26 %.

### Beispiel 6

2 444 Gew.-T. des NCO-Präpolymeren aus 2 000 Gew.-T. eines linearen Polyetherdiols (OH-Z: 56mg KOH/g) mit 444 Gew.-T. IPDI wurden bei 80 °C mit 310 Gew.-T. TAA gemäß Beispiel 1 umgesetzt. Die Viskosität des Reaktionsproduktes (bei RT) betrug 8 100 mPa·s; der blockierte NCO-Gehalt betrug 3 %.

### Beispiel 7

1 020 Gew.-T. eines verzweigten Polyoxypropylenglykols (OH-Zahl 27,5 mg KOH/g) und 111 Gew.-T. IPDI wurden bei 80 °C so lange erhitzt, bis der NCO-Gehalt auf 1,7 % gefallen war. Danach wurden 77,5 Gew.-T. TAA unter intensiver Rührung zugegeben und so lange bei 80 °C (unter N₂-Abdeckung) weiter erhitzt, bis der NCO-Gehalt auf < 0,3 % gefallen war. Das Reaktionsprodukt hatte (bei RT) eine Viskosität von 12 600 mPa·s und einen blockierten NCO-Gehalt von 1.6 %.

### Beispiel 8

3 666 Gew.-T. eines NCO-Präpolymeren aus 3 000 Gew.-T. eines verzweigten Polyethertriols (OH-Z: 56 mg KOH/g) und 666 Gew.-T. IPDI wurden bei 80 °C mit 465 Gew.-T. TAA entsprechend Beispiel 1 umgesetzt. Die Viskosität des Reaktionsproduktes betrug (bei RT) 9 600 mPa·s; der blockierte NCO-Gehalt lag bei 3 %.

### Beispiel 9

133 Gew.-T. des IPDI-Harnstoff-Addukts mit 31,5 % NCO, das aus 5,4 mol IPDI + 1 mol H₂O hergestellt wurde, wurden in 288 Gew.-T. Chlorparaffin mit 155 Gew.-T. TAA entsprechend Beispiel 1 umgesetzt. Das Reaktionsprodukt hatte (bei RT) eine Viskosität von 510 000 mPa·s und einen blockierten NCO-Gehalt von 7,2 %.

### Beispiel 10

1 094 Gew.-T. eines NCO-Präpolymeren aus 444 Gew.-T. IPDI und 650 Gew.-T. eines Polytetrahydrofurandiols (MG: 650) wurden in 1 404 Gew.-T. DISFLAMOLL^{(R)} TKP (Trikresylphosphat) mit 310 Gew.-T. TAA entsprechend Beispiel 1 umgesetzt. Das Reaktionsprodukt hatte (bei RT) eine Viskosität von 2 800 mPa·s und einen NCO-Gehalt von 2,9 %.

### Beispiel 11

1 444 Gew.-T. eines NCO-Präpolymeren aus 444 Gew.-T. IPDI und 1 000 Gew.-T. eines Polytetrahydrofurandiols (MG: 1 000) wurden in 1 754 Gew.-T. MESAMOLL^{(R)} (Alkylsulfonsäureester des Phenols) mit 310 Gew.-T. TAA entsprechend Beispiel 1 umgesetzt. Das Reaktionsprodukt hatte (bei RT) eine Viskosität von 2 400 mPa·s und einen blockierten NCO-Gehalt von 2,4 %.

### Beispiel 12

243 Gew.-T. eines Uretdiongruppen enthaltenden IPDI-Adduktes mit 17,3 % NCO wurden in 265 Gew. -T. MESAMOLL^{(R)} (Alkylsulfonsäureester des Phenols) mit 155 Gew.-T. TAA entsprechend Beispiel 1 umgesetzt. Das Reaktionsprodukt hatte (bei RT) eine Viskosität von 18 000 mPa·s und einen blockierten NCO-Gehalt von 6,3 %.

### Beispiel 13

800 Gew.-T. eines IPDI-Trimethylolpropanaddukts mit 15,7 % NCO wurden in 843 Gew.-T. MESAMOLL^{(R)} (Alkylsulfonsäureester des Phenols) mit 465 Gew.-T. TAA entsprechend Beispiel 1 umgesetzt. Das Reaktionsprodukt hatte (bei RT) eine Viskosität von 84 000 mPa·s und einen blockierten NCO-Gehalt von 5,9 %.

### Beispiel 14

195 Gew.-T. des trimeren Hexamethylendiisocyanats (Isocyenurat des Hexamethylendiisocyanats) wurden in einem Gemisch aus 146 Gew.-T. SOLVESSO^{(R)} 100 (Aromatengemisch, Siedegrenzen 163-181 °C) und 87 Gew.-T. Butylacetat mit 155 Gew.-T. TAA entsprechend Beispiel 4 umgesetzt. Das Reaktionsprodukt hatte (bei RT) eine Viskosität von 870 mPa·s und einen blockierten NCO-Gehalt von 7,2 %.

### Beispiel 15

178 Gew.-T. des entsprechend Beispiel 3 der DE-OS 23 08 015 hergestellten Biurets des Hexamethylendiisocyanats mit einem NCO-Gehalt von 23,6 % wurden in einem Gemisch aus 139 Gew.-T. SOLVESSO^{(R)} 100 (Aromatengemisch, Siedegrenzen 163-181 °C) und 83 Gew.-T. Butylacetat mit 155 Gew.-T. TAA entsprechend Beispiel 4 umgesetzt. Das Reaktionsprodukt hatte (bei RT) eine Viskosität von 2 160 mPa·s und einen blockierten NCO-Gehalt von 7,5 %.

### Beispiel 16

1 332 Gew.-T. IPDI wurden mit 134 Gew.-T. Trimethylolpropan in 1 907 Gew.-T. DISFLAMOLL^{(R)} TKP (Trikresylphsophat) so lange bei 80 °C erhitzt, bis der NCO-Gehalt der Lösung 11,2 % erreicht hatte. Anschließend wurde das Reaktionsgemisch mit 1 395 Gew.-T. TAA entsprechend Beispiel 1 umgesetzt. Das Reaktionsprodukt hatte (bei RT) eine Viskosität von 21 500 mPa·s und einen blockierten NCO-Gehalt von 7,9 %.

### Beispiel 17

444 Gew.-T. IPDI wurden mit 1 000 Gew.-T. eines Polytetramethylenglykols mit einem Molgewicht von ca. 1 000 in 1 839 Gew.-T. MESAMOLL^{(R)} (Alkylsulfonsäureester des Phenols) so lange bei 80 °C erhitzt, bis der NCO-Gehalt der Lösung 2,6 % erreicht hatte. Anschließend wurde das Reaktionsgemisch mit 155 Gew.-T. TAA und 240 Gew.-T. Sebacinsäureester des 4-Hydroxy-2.2.6.6-tetramethylpiperidins entsprechend Beispiel 1 umgesetzt. Das Reaktionsprodukt hatte (bei RT) eine Viskosität von 5 300 mPa·s und einen blockierten NCO-Gehalt von 2,2 %.

### Beispiel 18

444 Gew.-T. IPDI wurden mit 1 000 Gew.-T. eines Polytetramethylenglykols mit einem Molgewicht von ca. 1 000 in 1 924 Gew.-T. MESAMOLL^{(R)} (Alkylsulfonsäureester des Phenols) so lange bei 80 ° C erhitzt, bis der NCO-Gehalt der Lösung 2,5 % betrug. Anschließend wurde das Reaktionsgemisch mit 480 Gew.-T. Sebacinsäureester des 4-Hydroxy-2.2.6.6-tetramethylpiperidins entsprechend Beispiel 1 umgesetzt. Das Reaktionsprodukt hatte (bei RT) eine Viskosität von 13 000 mPa·s und einen blockierten NCO-Gehalt von 2,1 %.

### Beispiel 19

222 Gew.-T. IPDI wurden mit 812 Gew.-T. N-(2.2.6.6-Tetramethyl-4-piperidyl)-n-dodecyl-succinimid (Beispiel 1, DE-OS 30 06 272) in 1 034 Gew.-T. MESAMOLL^{(R)} (Alkylsulfonsäureester des Phenols) entsprechend Beispiel 1 umgesetzt. Das Reaktionsprodukt hatte (bei RT) eine Viskosität von 2 800 mPa·s und einen blockierten NCO-Gehalt von 4 %.

### Beispiel 20

222 Gew.-T. IPDI wurden mit 368 Gew.-T. 4-Dimethylamino-2.2.6.6-tetramethylpiperidin entsprechend Beispiel 1 umgesetzt. Das Reaktionsprodukt hatte einen Schmelzbereich von 60 bis 64 °C und einen blockierten NCO-Gehalt von 14,2 %.

## Patentansprüche

1. Blockierte aliphatische, cycloaliphatische, araliphatische oder arylsubstituierte aliphatische Polyisocyanate, wobei diese ggf. mit Polyolen kettenverlängert sein können und die Polyisocyanate auch Uretdion-, Carbodiimid-, Harnstoff- und Isocyanurat bzw. Biuretgruppen aufweisen können, enthaltend als Blockierungsmittel sterisch gehinderte Piperidin-Derivate der Formeln A oder B oder der Formel C 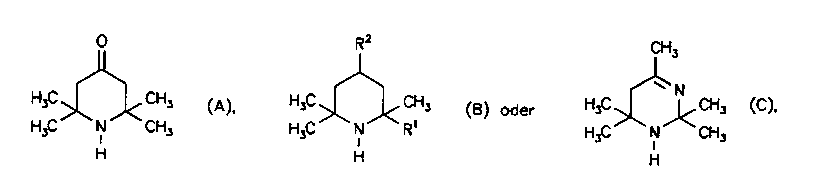 wobei
R¹ = H, CH₃,
R² = (-O-)ₙR³, (-O-CO)ₙ-R³, -OH , -NH-CO-R³,
-N-(CH₃)₂ , 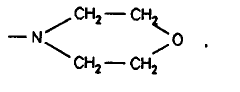 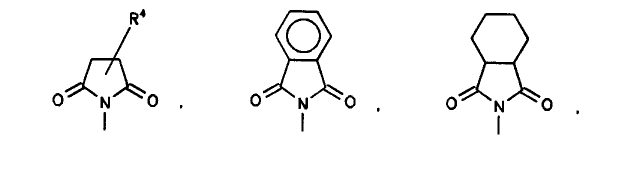
n = 1,2
R³ = C₁-C₁₈-Alkyl, wenn n = 1 und
R³ = C₂-C₁₈-Alkylen, wenn n = 2
R⁴ = H, C₁-C₂₀-Alkyl bedeuten, und
wobei das NCO:NH-Verhältnis 0,5 bis 1,4, vorzugsweise 0,9 bis 1,1, insbesondere 1, beträgt.

2. Blockierte Polyisocyanate nach Anspruch 1,
dadurch gekennzeichnet,
daß das Blockierungsmittel 2.2.6.6-Tetramethylpiperidinon-4 ist.

3. Blockierte Polyisocyanate nach den Ansprüchen 1 bis 2,
dadurch gekennzeichnet,
daß dimere oder trimere aliphatische, cycloaliphatische, araliphatische oder arylsubstituierte aliphatische Polyisocyanate als Ausgangsverbindungen eingesetzt werden.

4. Blockierte Polyisocyanate nach Anspruch 3,
dadurch gekennzeichnet,
daß 1.6-Hexamethylendiisocyanat, 2-Methyl-pentamethylendiisocyanat, 2.2.4(2.4.4)-Trimethyl-hexamethylendiisocyanat (TMDI), 1.12-Dodecandiisocyanat, omega,omega'-Diisocyanatodipropylether, Isophorondiisocyanat (IPDI), 1.4-Cyclohexandiisocyanat, 1.4-Diisocyanatomethylcyclohexan, (m,p)-Tetramethylxylylendiisocanat (m,p-TMXDI), eingesetzt wird.

## Claims

1. Blocked aliphatic, cycloaliphatic, araliphatic or aryl-substituted aliphatic polyisocyanates which may have been extended with polyols and which may also have uretdione, carbodiimide, urea and isocyanurate and/or biuret groups, containing as blocking agents sterically hindered piperidine derivatives of the formula A or B or of the formula C 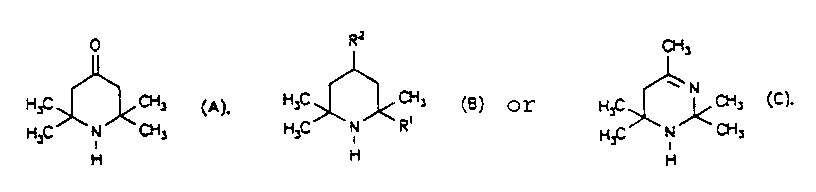 where
R¹ = H or CH₃,
R² = (-O-)ₙR³, (-O-CO)ₙ-R³, -OH, -NH-CO-R^{3,} -N-(CH₃)₂, 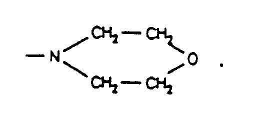 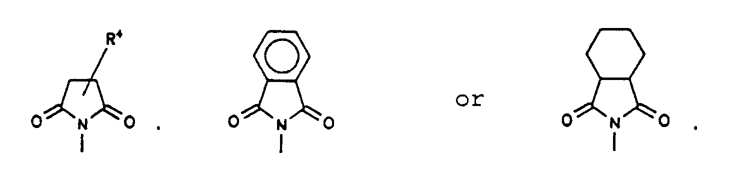
n = 1 or 2
R³ = C₁-C₁₈-alkyl if n = 1 and
R³ = C₂-C₁₈-alkylene if n = 2,
R⁴ = H or C₁-C₂₀-alkyl, and
the NCO:NH ratio is from 0.5 to 1.4, preferably from 0.9 to 1.1, in particular 1.

2. Blocked polyisocyanates according to claim 1, characterised in that the blocking agent is 2,2,6,6-tetramethylpiperidin-4-one.

3. Blocked polyisocyanates according to either of claims 1 and 2, characterised in that dimeric or trimeric aliphatic, cycloaliphatic, araliphatic or aryl-substituted aliphatic polyisocyanates are employed as starting compounds.

4. Blocked polyisocyanates according to claim 3, characterised in that 1,6-hexamethylene diisocyanate, 2-methylpentamethylene diisocyanate, 2,2,4(2,4,4)-trimethylhexamethylene diisocyanate (TMDI), 1,12-dodecane diisocyanate, omega,omega'-diisocyanatodipropyl ether, isophorone diisocyanate (IPDI), 1,4-cyclohexane diisocyanate, 1,4-diisocyanatomethylcyclohexane or (m,p)-tetramethylxylylene diisocyanate (m,p-TMXDI) is employed.

## Revendications

1. Polyisocyanates aliphatiques, cycloaliphatiques, araliphatiques, ou aliphatiques substitués par un aryle, bloqués dans lesquels ceux-ci peuvent être le cas échéant soumis à une prolongation de chaîne par des polyols et les polyisocyanates peuvent aussi posséder des groupes uretdione, carbodiimide, urée et isocyanurate ou biuret, contenant comme agent de blocage des dérivés de pipéridine empêchés stériquement des formules A ou B ou de formule C : 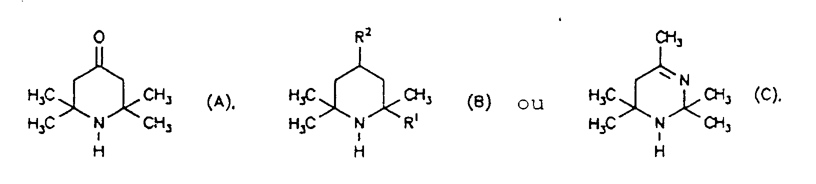 dans lesquelles
R¹ = H, CH₃,
R¹ = (-O-)ₙR³, (-O-CO)ₙ-R³, -OH , -NH-CO-R³, -N-(CH₃)₂ 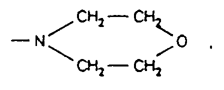 , 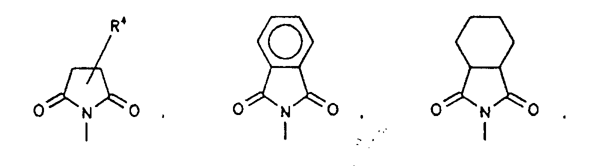
n = 1,2
R³ = Alkyle en C₁-C₁₈, quand n = 1 et
R³ = Alkylène en C₂-C₁₈, quand n = 2
R⁴ = H, Alkyle en C₁-C₂₀ et
dans lesquelles le rapport NCO/NH s'élève à 0,5 à 1,4 de préférence 0,9 à 1,1 en particulier à 1.

2. Polyisocyanates bloqués selon la revendication 1,
caractérisés en ce que
l'agent de blocage est la 2,2,6,6-tétraméthylpiperidinone-4.

3. Polyisocyanates bloqués selon les revendications 1 à 2,
caractérisés en ce que
des polyisocyanates dimères ou trimères aliphatiques cycloaliphatiques, araliphatiques ou aliphatiques substitués par un aryle, sont mis en oeuvre comme composés de départ.

4. Polyisocyanates bloqués selon la revendication 3,
caractérisés en ce qu'
on met en oeuvre le 1,6-hexaméthylènediisocyanate, le 2-méthylpentaméthylènediisocyanate, le 2,2,4(2,4,4)-triméthylhexaméthylènediisocyanate (TMDI), le 1,12-dodécanediisocyanate,l'omega-omega'-diisocyanatodipropyléther, l'isophoronediisocyanate (IPDI), le 1,4-cyclohexanediisocyanate, le 1,4-diisocyanatométhylcyclohexane, le (m,p.)-tétraméthylxylylènediisocyanate (m,p-TXMDI).
